# EUROPEAN PATENT SPECIFICATION

(11) **EP 0 943 344 B1**
(45) Date of publication and mention of the grant of the patent: **04.09.2002**
(21) Application number: 99105678.9
(22) Date of filing: 19.03.1999
(51) Int. Cl.: A61L 9/03, A01M 1/20

(54) **Vaporizer for a liquid insecticide or perfume composition, with regulation of the intensity of the heat flow**
Verdampfer für ein flüssiges Insektizid oder eine Parfümzusammensetzung mit regulierbarem Wärmeübergang
Vaporisateur pour un insecticide liquide ou une composition parfumante avec réglage du flux de chaleur

(30) Priority: 19.03.1998 IT MI980554
(43) Date of publication of application: 22.09.1999
(73) Proprietor: ZOBELE HOLDING S.P.A., 38100 TRENTO (IT)
(72) Inventor: Zobele, Franco, 38100 Trento (IT)
(74) Representative: Faggioni, Marco, Dr. Ing.

(56) References cited:
- WO-A-98/58692
- US-A- 3 872 280
- US-A- 5 647 053

## Description

The present invention relates to a vaporizer for a liquid insecticide or perfume composition, provided with a system for regulating the intensity of the heat flow which is transmitted by the heating element to the wick of the container holding the liquid composition. The vaporizers to which the present invention applies are those of the type which are provided with a rotatable plug - so as to allow use of the apparatus both with sockets having horizontal holes and with sockets having vertical holes - and in which the electric heating element is integral with the plug and rotates together therewith.

Vaporizers for liquid insecticide and perfume composition comprising devices for regulating the intensity of evaporation of the active substance are already present on the market. These devices are based either on the use of special circuits for regulating the intensity of the current supplied to the electric heating element or on the use of mechanical devices which allow the wick and the heating element to be moved towards or away from each other.

None of these known regulating devices, however, may be used with success in the vaporizers of the present invention, since in these latter the heating element is not fixed to the housing, but to the rotatable plug. On the one hand, in fact, any electric circuits for regulating the current intensity cannot be easily and securely housed in the plug, for obvious reasons of shortage of space and owing to the fact that these circuits would be extremely close to the heating element; on the other hand, the mechanical regulating devices of the known type envisage coaxial relative displacement of the heating element and wick, which would be entirely unfeasible in the case of vaporizers according to the present invention in which the rotating plug is always arranged laterally with respect to the wick. Document US5647053 discloses a vaporizer with the features of the premble of claim 1.

The object of the present invention is therefore that of providing a vaporizer of the type mentioned above, in which it is also possible to obtain, by means of a simple and reliable structure, minimum/maximum type regulation of the heat flow received by the wick.

This object is achieved, according to the present invention, by means of a vaporizer for liquid insecticide or perfume composition, consisting of a housing supporting the liquid composition container and confining the wick thereof, and of a plug body to which an electric heating device is associated, said body being rotatable between at least one vertical plug position and at least one horizontal plug position, characterized in that the means for rotatably supporting the plug body on the housing allow, in addition to rotatably degree of freedom of the plug, at least one other degree of freedom of the plug in a direction perpendicular to the plane of rotation of the plug or in a direction lying on said plane.

According to a first embodiment of the vaporizer, said plug body has a cylindrical base mating with a corresponding cylindrical cavity of the housing, two conjugate grooves being formed on the side surface of said base, said grooves being apt to co-operate with corresponding pins provided in said cylindrical cavity, so as to allow axial displacements of the plug body during rotation thereof.

In a second embodiment of the vaporizer, said plug body is engaged in an eccentric and freely rotatable manner inside a disk element having an axis of rotation parallel to that of the plug body, said disk element being in turn engaged in a freely rotatable manner with said housing, so as to allow transverse displacements of the plug body during rotation of said disk element.

The invention will now be more clearly described in detail, with reference to the accompanying drawings which show preferred embodiments thereof and in which:
Fig. 1 is an exploded side view of a first embodiment of a vaporizer according to the present invention, without container;
Fig. 2 is an exploded plan view of the vaporizer according to Fig. 1;
Fig. 3 is a rear elevation view of the rotatable plug body of the vaporizer according to Fig. 1;
Fig. 4 is a front elevation view of the housing of the vaporizer according to Fig. 1;
Fig. 5 is a front view of a second embodiment of a vaporizer according to the present invention, without the container;
Fig. 6 is a view similar to that of Fig. 3 of a second operating position of the vaporizer; and
Fig. 7 is a front view of a variant of the second embodiment of the vaporizer according to the present invention.

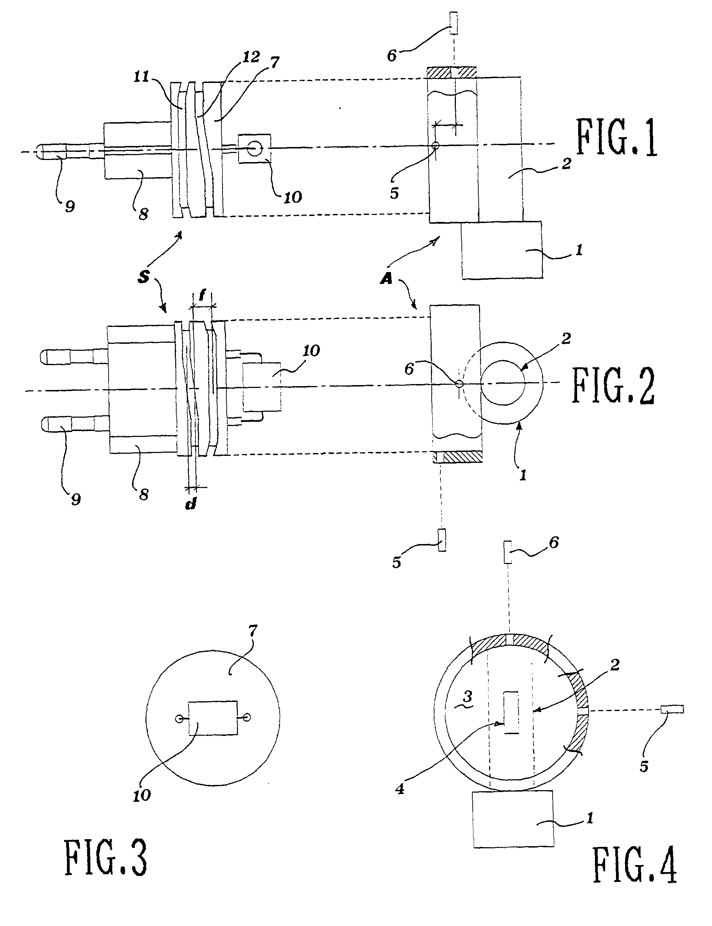

A first embodiment of the vaporizer according to the present invention is shown in Figs. 1 to 4, in which it can be clearly seen how the vaporizer consists of a housing A and a rotatable plug body S snugly fitting into a corresponding cavity of the housing A.

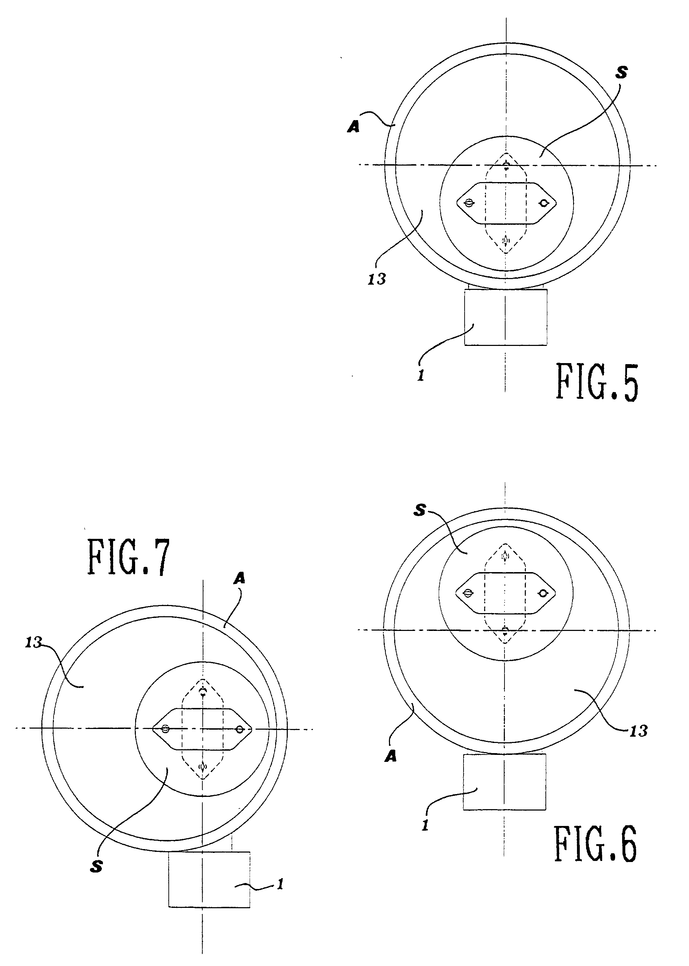

The housing A, which is shown in a very schematic way, comprises a cylindrical bush 1 which is threaded internally and arranged in the bottom portion of the housing. The threaded neck of a container holding the liquid to be vaporized (not shown), can be engaged, in a known manner, into the bush 1. The bush 1 is connected at the top to a hollow cylindrical element 2, apt to protect and receive the wick which protrudes from the container holding the liquid. At the front the housing A has a cylindrical chamber 3 intended to receive the rotatable plug body S, as will be explained in greater detail below. The end wall of the chamber 3 has an opening 4 (shown in Fig. 4) which connects the chamber itself to the inside of the cylindrical element 2. The cylindrical side wall of the chamber 3 is provided with at least two through holes, which are offset both angularly, for example by an angle of 90° or 180°, and longitudinally, by a distance f, and which are intended to receive corresponding pins 5 and 6.

The rotatable plug body S consists of a cylindrical base 7 integral with a plug portion 8 provided with connector pins 9. Metal leads connected to the connector pins 9 pass through the plug body and emerge on the opposite side thereof where they are welded, or in any other way connected, to an electric heating element 10, for example consisting of a ceramic block having an electric resistance cemented therein. Obviously heating elements of different types and different shapes may be used, for example posistors (known as "PTC"), which all fall within the scope of the invention. The cylindrical base of the 7 of the plug body S has a sufficient height to allow the formation of two annular grooves 11 and 12 which extend as a closed rings over the whole side surface thereof. The profile of each groove 11 or 12 preferably comprises two half-rings which are longitudinally offset by a predefined distance d and are connected by inclined sections. The profiles of the two grooves 11 and 12 are identical, but angularly offset by the same angle as the angle by which the holes receiving the pins 5 and 6 are offset. In the drawings, the pins 5 and 6 are offset by 90° and the profiles of the two grooves 11 and 12 are therefore offset by the same angle. Here and below, the expression "angularly offset" refers always to angles measured with respect to the axis of rotation of the rotatable plug body S, said axis being indicated by dot-dash lines in Figs. 1 and 2, while the expression "longitudinally offset" always refers to distances measured along said axis.

During assembly of the vaporizer, the rotatable plug body S is inserted into the housing A so that its cylindrical base 7 engages snugly, in a freely rotatable manner, within the cylindrical chamber 3. The pins 5 and 6 are then inserted and blocked into the respective holes so that they protrude into the cavity 3, i.e. respectively into the grooves 11 and 12. For a better connection between the plug body S and the housing A, the cross-section of the grooves 11 and 12 is identical to that of the pins 5 and 6, except that there is a slight degree of slack between the parts which allows the plug body S to be rotated without unduly high friction, while the pins 5 and 6 slide inside the grooves 11 and 12. This particular connection leaves the plug body S completely free to rotate, while at the same time it guides it along the profile of the grooves 11 and 12. This is made possible by the fact that the two grooves 11 and 12 are longitudinally offset by the same amount f as the amount f by which the pins 5 and 6 are longitudinally offset and that the angular offset arrangement of said pins - thanks to which an adequate stability of the plug body with respect to flexural forces is obtained - is identical to the angular offset arrangement of the profiles of said grooves. The pins 5 and 6 may obviously be formed as one piece with the housing A, by suitably modifying the base 7 of the plug body S so as to allow insertion of the said pins into the corresponding grooves 11 and 12.

Owing to this connection, therefore, the plug body S, by rotating with respect to the housing A, assumes a first longitudinal position during 180° of rotation and a second longitudinal position, offset with respect to the preceding position by the distance d, during the other 180° of rotation. In a longitudinal position, therefore, the heating element 10 will be close to and preferably adherent to the opening 4 and the wick housed in the cylindrical element 2 will therefore receive a maximum flow of heat; in the other position, on the contrary, the heating element will be situated at a distance d from said opening 4 and the wick will therefore receive a minimum flow of heat, thus achieving the object of the invention of obtaining, in a simple, reliable and low-cost manner, the possibility of regulation of the heat flow received by the wick.

It should be noted also that, since the two longitudinal positions of the plug each remain unchanged during 180° rotation of the plug (i.e. over the angular extension of one of the two half-rings from which each of the grooves 11 and 12 is formed), each of said positions is available both with the connector pins 9 in the horizontal and in the vertical position.

Figs. 5, 6 and 7 show a second embodiment of the invention, in which the plug body S has the possibility of moving transversely in its plane of rotation, instead of, as in the preceding embodiment, longitudinally along its axis of rotation. As can be seen, in fact, in Fig. 5, the plug body S is housed, freely rotatably, inside a disk element 13, in turn housed, in a freely rotating manner, inside the housing A. The plug body S is inserted in the disk element in an eccentric position such that, upon rotation of said element, the plug body assumes different lateral positions with respect to the axis of the wick, shown in vertical dot-dash lines in Figs. 5 to 7. The type of rotatable connection between the plug body S and the disk element 13 and between the latter and the housing A does not need to be described in detail since it consists of a connection of the groove and rib type, which is well-known per se in that it is identical to that used for connecting the rotatable plug body to the housing in conventional vaporizers.

With this particular type of structure of the vaporiser, the plug body S is able to assume a variety of positions which are offset horizontally or vertically with respect to the wick, so as to allow the desired regulation of the heat flow which reaches the latter. For example, according to a variant in which the centre of the disk element 13 is aligned with the axis of the wick - namely its axis of rotation intersects the axis of the wick - as illustrated in Figs. 5 and 6, the plug body is able to assume the bottom position shown in Fig. 5, where the heat flow is at its maximum because the rising heat flow envelopes the wick over its entire length, or the high position illustrated in Fig. 6 where the heat flow is less because the rising heat current envelopes only the top part of the wick. The plug body S, finally, may be arranged also in the two end lateral positions (not shown) which it assumes during rotation of the disk element 13 and where the heating element 10 is located axially displaced with respect to the wick and therefore the heat flow is at its minimum.

As can be clearly seen from the drawings and moreover as can be understood from the design of this vaporizer, in each working position of the plug body S, the connector pins 9 may assume equally well the horizontal position (shown in solid lines) or the vertical position (shown in broken lines) so as to be adaptable to the position of the socket into which it must be inserted. Snap-engagement stop elements, which are well known per se, may be envisaged so as to provide the user with a reliable reference point for the correct working positions of the plug body S and the disk element 13.

In another variant of the second embodiment described above, the disk element 13 is mounted on the housing A, eccentric with respect to the wick, and in particular the axis of the plug body S intersects the axis of the connector pins in one of the end lateral positions assumed by the plug body S during rotation of the disk element 13, as shown in Fig. 7. When the vaporiser has this type of structure, the wick will receive the maximum heat flow in the position shown in Fig. 7, the minimum heat flow when the plug body S is in the diametrically opposite position of the disk element 13, and an intermediate heat flow, and specifically a greater heat flow in a lower position and a smaller heat flow in a high position, when the plug body S is located aligned respectively underneath and above the centre of the disk element 13 (positions similar to those shown in Figs. 5 and 6).

From the above description it is clear how the vaporizer according to the present invention has fully achieved the desired objects. It offers, in fact, in a vaporizer having a simple and sturdy structure, the possibility for easy regulation of the heat flow which reaches the wick, while maintaining all the advantages of the vaporizers with a rotatable plug body and heating element fixed to said plug body.

Obviously the invention has been described with reference to particular embodiments thereof, but it is clear that the scope of the invention is not limited to these embodiments, which must be regarded therefore as being purely an example of any type of support means which allows the rotatable plug body S to be joined to the housing A, ensuring, in addition to the rotational degree of freedom of at least one further degree of transverse or axial freedom for the plug body, in accordance with the accompanying claims.

## Claims

1. Vaporizer for liquid insecticide or perfume composition, consisting of a housing (A) supporting the liquid composition container and confining the wick thereof, and of a plug body (S) to which an electric heating device (10) is associated, said body (S) being rotatable between at least one vertical plug position and at least one horizontal plug position, **characterized in that** the means for rotatably supporting the plug body (S) on the housing (A) allow, in addition to rotatably degree of freedom of the plug (S), at least one other degree of freedom of the plug (S) in a direction perpendicular to the plane of rotation of the plug (S) or in a direction lying on said plane.

2. Vaporizer as claimed in Claim 1, in which said plug body (S) has a cylindrical base (7) for engagement with a corresponding cylindrical cavity (3) of the housing (A), there being formed on the side surface of said base two conjugate grooves (11,12) which are identical and offset longitudinally and angularly and are able to co-operate with corresponding pins (5,6) provided in said cylindrical cavity (3) so as to allow axial displacements of the plug body (S) during rotation thereof.

3. Vaporizer as claimed in Claim 2, in which the profile of both said grooves (11,12) consists of two half-rings which are offset longitudinally and connected by inclined sections.

4. Vaporizer as claimed in Claim 2, in which said pins (5,6) of the cylindrical cavity (3) are angularly offset with respect to each other at an angle equal to the angle with which said conjugate grooves (11,12) are offset.

5. Vaporizer as claimed in Claim 4, in which said offset angle ranges between 90° and 180°.

6. Vaporizer as claimed in Claim 2, in which said pins (5,6) of the cylindrical cavity (3) are offset longitudinally with respect to each other by a distance (f) equal to the distance by which said conjugate grooves (11,12) are longitudinally offset.

7. Vaporizer as claimed in Claim 1, in which said plug body (S) is engaged in an eccentric and freely rotatable manner inside a disk element (13) having an axis of rotation parallel to that of the plug body (S), said disk element (13) being in turn engaged in a freely rotatable manner with said housing (A) so as to allow transverse displacements of the plug body (S) during rotation of said disk element (13).

8. Vaporizer as claimed in Claim 7, in which the axis of rotation of said disk element (13) intersects the axis of the wick.

9. Vaporizer as claimed in Claim 7, in which the axis of rotation of the plug body (S) intersects the axis of the wick in one of the two lateral positions which the plug body (S) assumes during rotation of the disk element (13).

## Patentansprüche

1. Verdampfer für ein flüssiges Insektizid oder eine Parfumzusammensetzung, bestehend aus einem Gehäuse (A), welches den Behälter der flüssigen Zusammensetzung trägt und den Docht desselben einschließt, und einem Steckkörper (S), an welchem eine elektrische Heizvorrichtung (10) assoziiert ist, wobei der Körper (S) zwischen wenig-. stens einer vertikalen Steckerposition und wenigstens einer horizontalen Steckerposition drehbar ist, **dadurch gekennzeichnet, daß** das Mittel zum drehbaren Tragen des Steckkörpers (S) auf dem Gehäuse (A) zusätzlich zu einem Drehfreiheitsgrad des Steckers (S) wenigstens einen weiteren Freiheitsgrad des Steckers (S) in einer Richtung senkrecht zu der Drehebene des Steckers (S) oder in einer Richtung, die in besagter Ebene liegt, ermöglicht.

2. Verdampfer nach Anspruch 1, bei dem der Steckkörper (S) eine zylinderförmige Grundfläche (7) zum Eingriff mit einem korrespondierenden zylinderförmigen Hohlraum (3) des Gehäuses (A) aufweist, wobei auf der seitlichen Oberfläche der Grundfläche zwei konjugate Rillen (11, 12) gebildet sind, welche identisch sind und der Länge nach und winkelig gegeneinander versetzt sind und in der Lage sind, mit korrespondierenden Stiften (5, 6) zu kooperieren, welche in dem zylinderförmigen Hohlraum (3) bereitgestellt sind, um so axiale Verschiebungen des Steckkörpers (S) während einer Rotation desselben zu ermöglichen.

3. Verdampfer nach Anspruch 2, bei dem das Profil der beiden Rillen (11, 12) aus zwei Halbringen besteht, welche der Länge nach gegeneinander versetzt und durch geneigte Abschnitte verbunden sind.

4. Verdampfer nach Anspruch 2, bei dem die Stifte (5, 6) des zylinderförmigen Hohlraums (3) winkelig zueinander mit einem Winkel versetzt sind, welcher gleich ist dem Winkel, mit dem die konjugaten Rillen (11, 12) gegeneinander versetzt sind.

5. Verdampfer nach Anspruch 4, bei dem der Versatzwinkel in einem Bereich zwischen 90° und 180° liegt.

6. Verdampfer nach Anspruch 2, bei dem die Stifte (5, 6) des zylinderförmigen Hohlraums (3) der Länge nach zueinander mit einem Abstand (F) versetzt sind, welcher gleich ist dem Abstand, mit dem die konjugaten Rillen (11, 12) der Länge nach gegeneinander versetzt sind.

7. Verdampfer nach Anspruch 1, bei dem der Steckkörper (S) auf eine exzentrische und frei drehbare Art und Weise innerhalb eines Scheibenelements (13) in Eingriff ist, welches eine Drehachse parallel zu derjenigen des Steckkörpers (S) aufweist, wobei das Scheibenelement (13) wiederum auf eine frei drehbare Art und Weise mit dem Gehäuse (A) in Eingriff ist, um 10 transversale Verschiebungen des Steckkörpers (S) während einer Rotation des Scheibenelements (13) zu ermöglichen.

8. Verdampfer nach Anspruch 7, bei dem die Drehachse des Scheibenelements (13) die Achse des Dochts kreuzt.

9. Verdampfer nach Anspruch 7, bei dem die Drehachse des Steckkörpers (S) die Achse des Dochts in einer der zwei Seitenpositionen kreuzt, welche der Steckkörper (S) während einer Rotation des Scheibenelements (13) annimmt.

## Revendications

1. Vaporisateur pour composition liquide insecticide ou parfumée, consistant en un boîtier (A) supportant le réservoir de composition liquide et enfermant sa mèche, et en un corps de prise de courant (S) auquel un dispositif de chauffage électrique (10) est associé, ledit corps (S) étant adapté à tourner entre au moins une position de prise de courant verticale et au moins une position de prise de courant horizontale, **caractérisé en ce que** les moyens pour supporter en rotation le corps de prise de courant (S) sur le boîtier (A) permettent, outre un degré de liberté en rotation de la prise de courant (S), au moins un autre degré de liberté de la prise de courant (S) dans une direction perpendiculaire au plan de rotation de la prise de courant (S) ou dans une direction se situant sur ledit plan.

2. Vaporisateur selon la revendication 1, dans lequel ledit corps de prise de courant (S) comporte une base cylindrique (7) pour engagement avec une cavité cylindrique correspondante (3) du boîtier (A), sur la surface latérale de ladite base étant formées deux rainures conjuguées (11, 12) qui sont identiques et décalées longitudinalement et angulairement et sont adaptées à coopérer avec des tiges correspondantes (5, 6) prévues dans ladite cavité cylindrique (3) de manière à permettre des déplacements axiaux du corps de prise de courant (S) durant sa rotation.

3. Vaporisateur selon la revendication 2, dans lequel le profil desdites deux rainures (11, 12) consiste en deux demi-anneaux qui sont décalés longitudinalement et reliés par des sections inclinées.

4. Vaporisateur selon la revendication 2, dans lequel lesdites tiges (5, 6) de la cavité cylindrique (3) sont décalées l'une de l'autre d'un angle égal à l'angle duquel lesdites rainures conjuguées (11, 12) sont décalées.

5. Vaporisateur selon la revendication 4, dans lequel ledit angle de décalage est compris entre 90° et 180°.

6. Vaporisateur selon la revendication 2, dans lequel lesdites tiges (5, 6) de la cavité cylindrique (3) sont décalées l'une de l'autre longitudinalement d'une distance (F) égale à la distance de laquelle lesdites rainures conjuguées (11, 12) sont décalées longitudinalement.

7. Vaporisateur selon la revendication 1, dans lequel ledit corps de prise de courant (S) est engagé de manière excentrique et en rotation libre à l'intérieur d'un élément formant disque (13) comportant un axe de rotation parallèle à celui du corps de prise de courant (S), ledit élément formant disque (13) étant engagé à son tour en rotation libre avec ledit boîtier (A) de manière à permettre des déplacements transversaux du corps de prise de courant (S) durant la rotation dudit élément formant disque (13).

8. Vaporisateur selon la revendication 7, dans lequel l'axe de rotation dudit élément formant disque (13) coupe l'axe de la mèche.

9. Vaporisateur selon la revendication 7, dans lequel l'axe de rotation du corps de prise de courant (S) coupe l'axe de la mèche dans l'une des deux positions latérales que le corps de prise de courant (S) adopte durant la rotation de l'élément formant disque (13).
